# EUROPEAN PATENT SPECIFICATION

(11) **EP 4 028 991 B1**
(45) Date of publication and mention of the grant of the patent: **12.08.2026**
(21) Application number: 20772174.7
(22) Date of filing: 02.09.2020
(51) Int. Cl.: G06T 5/50, G06T 7/00

(54) **CONFIDENCE MAP FOR RADIOGRAPHIC IMAGE OPTIMIZATION**
KONFIDENZKARTE FÜR DIE OPTIMIERUNG VON RÖNTGENBILDERN
CARTE DE CONFIANCE POUR OPTIMISATION D'IMAGE RADIOGRAPHIQUE

(30) Priority: 10.09.2019 US 201962898019 P
(43) Date of publication of application: 20.07.2022
(73) Proprietor: Midea Imaging Singapore Pte. Ltd., Singapore 069545 (SG)
(72) Inventor: VOGELSANG, Levon, O., Rochester, NY 14608 (US); WANG, Xiaohui, Rochester, NY 14608 (US); YORKSTON, John, Rochester, NY 14608 (US); SEHNERT, William, J., Rochester, NY 14608 (US)
(74) Representative: Ran, Handong
(86) International application number: PCT/US2020/049008
(87) International publication number: WO 2021/050332

(56) References cited:
- US-A- 5 768 405
- ANDREAS RICK ET AL: "Quantitative Modelling of Microcalcification Detection in Digital Mammography", 1 January 2006, MEDICAL IMAGE COMPUTING AND COMPUTER ASSISTED INTERVENTION - MICCAI '99 : SECOND INTERNATIONAL CONFERENCE, CAMBRIDGE, UK, SEPTEMBER 19 - 22, 1999; [LECTURE NOTES IN COMPUTER SCIENCE ; 1679], SPRINGER, BERLIN [U.A.], PAGE(S) 32 - 41, ISBN: 978-3-540-66503-8, XP019036183
- MASAKI MORI1 ET AL: "Method of measuring contrast-to-noise ratio (CNR) in nonuniform image area in digital radiography", ELECTRONICS AND COMMUNICATIONS IN JAPAN, SCRIPTA TECHNICA. NEW YORK, US, vol. 96, no. 7, 1 July 2013 (2013-07-01), pages 32 - 41, XP001584292, DOI: 10.1002/ECJ.11416

## Description

### BACKGROUND OF THE INVENTION

The disclosure relates generally to image processing, and in particular to medical image processing. More specifically, the disclosure relates to validation of image content for a processed image.

Advantages of digital radiography (DR) imaging and related digital imaging modalities for 2D and 3D DR imaging over earlier radiographic methods are widely acknowledged, including benefits such as rapid data acquisition and processing, networked and wireless delivery, and multiple options for display. Continuing advances in performance and usability of DR imaging cassettes make it possible for these detector devices to extend the utility of radiographic imaging to more portable imaging systems, for example, making radiographic imaging available for an expanded range of environments and patient conditions.

In order to provide images having suitable clinical and diagnostic value, DR and related digital systems routinely process the received image data at one or more levels. For typical DR systems, for example, raw digital data from the DR detector is initially pre-processed according to calibration data that is maintained for the individual detector and for the receiving system hardware. Other levels of image data processing follow this pre-processing step, executing algorithms intended to suppress noise content, adjust intensity or brightness and contrast of image features, adjust gain, identify and correct or suppress defects and otherwise adapt image presentation into a form suitable for viewing by the practitioner.

Numerous types of image processing have been devised for improving the accuracy and usefulness of the digital image data that has been obtained. Image processing may be local to a specific area in the image, for example, to compensate for pixels that are unresponsive or perform poorly. Other image processing routines can be more extensive, such as algorithms that perform globally across the image to improve visualization of features by enhancing or suppressing certain elements in the image. In many cases, the image processing activity lies outside of user control, although many systems provide post processing options for some of the processing.

Overall, the image processing techniques that are applied to the digitally captured image data may have varying degrees of sophistication; as computer power has increased, so too has the complexity of the algorithms used for conditioning the image content. One promising area for increased computational power and impact is the use of machine-learning algorithms that can be trained according to results of numerous exemplary images, following the response pattern of a skilled human observer. Compared against more conventional algorithmic approaches based on data analysis and processing, machine learning has advantages of rapid recognition and decision-making that emulate more complex pattern recognition and response capabilities of an experienced human observer.

As image processing methods become potentially more powerful and capable, however, practitioners are naturally cautious and can have some reservations with respect to fidelity to image content, particularly for images that may be used to aid in diagnosis of a patient's condition. It is possible that, in some cases, processing may not enhance the visibility of various features but may, in fact, make them more difficult to perceive or distinguish. Difficulties due to image processing can be particularly problematic where subtle changes in the condition of the imaged anatomy are indicative of a pathological condition and need to be clearly visible.

Suitable image processing can enhance presentation of the imaged anatomy; however, this enhancement must neither suppress image features that can be diagnostically relevant nor add image artifacts that can misrepresent the imaged anatomy. In response to this concern for accurate representation, image processing logic is carefully designed so that the resulting processed image faithfully represents the true data content of the imaged subject anatomy.

Thus, it can be appreciated that there would be significant value in an automated utility that can provide the viewing practitioner with an indication of the overall consistency of, and confidence in, image processing that has been applied to a particular digital radiographic image.

Further reference is made to Andreas Rick, et al.: "Quantitative Modelling of Microcalcification Detection in Digital Mammography", from the MICCAI 1999 SECOND INTERNATIONAL CONFERENCE, CAMBRIDGE, UK, pp. 32-42, published by Springer-Verlag, Berlin, 1999, describing a simulation framework for use in image acquisition on digital mammography systems. The framework is used to analyze the performance of a method for the detection of microcalcifications by a series of top-hat operators. The framework determines the theoretical number of false positives and true positives for a given set of acquisition parameters and size of the microcalcifications. A minimal size of microcalcifications that is detected with sufficient certitude is analyzed as a function of the pixel size of the detector and the acquisition conditions.

US 5 768 405 A describes a digital image processing method for determining the center and the width of objects in the form of contrasting bands on a background. The objects are in the form of contrasting bands of substantially uniform intensity on a substantially uniform background. Pixels situated on the central lines of the objects are identified, which is referred to as a "tracking" step. The method includes applying, to each image, a series of N lozenge-type-bidimensional, selective, recursive low-pass filters having respective ones of the principal directions angulary spaced apart 180°.

The discussion above is merely provided for general background information and is not intended to be used as an aid in determining the scope of the claimed subject matter.

### BRIEF DESCRIPTION OF THE INVENTION

In accordance with the present invention, a computer comprising a processing system and a computer implemented method for processing a digital radiographic image of a subject anatomy as set forth in Claims 1 and 8 is provided. Preferred embodiments of the invention are claimed in the dependent claims. In particular, a computer implemented method for processing a digital radiographic image captures and stores an unprocessed radiographic image acquired from a digital radiography (DR) detector. The image is processed and stored. The method combines the image processed radiographic image and the unprocessed radiographic image to form a residual image and digitally analyzes the residual image to determine a confidence rating of the residual image.

An object of the present disclosure is to advance the art of image processing, particularly for medical images, including digital radiographic images.

Another object of the present disclosure is to provide tools for evaluating changes in image content that can result from digital image processing.

These objects are given only by way of illustrative example, and such objects may be exemplary of one or more embodiments of the invention. Other desirable objectives and advantages inherently achieved may occur or become apparent to those skilled in the art. The invention is defined by the appended claims.

The summary descriptions above are not meant to describe individual separate embodiments whose elements are not interchangeable. In fact, many of the elements described as related to a particular embodiment can be used together with, and possibly interchanged with, elements of other described embodiments.

This brief description of the invention is intended only to provide a brief overview of subject matter disclosed herein according to one or more illustrative embodiments, and does not serve as a guide to interpreting the claims or to define or limit the scope of the invention, which is defined only by the appended claims. This brief description is provided to introduce an illustrative selection of concepts in a simplified form that are further described below in the detailed description. This brief description is not intended to identify key features or essential features of the claimed subject matter, nor is it intended to be used as an aid in determining the scope of the claimed subject matter. The claimed subject matter is not limited to implementations that solve any or all disadvantages noted in the background.

### BRIEF DESCRIPTION OF THE DRAWINGS

So that the manner in which the features of the invention can be understood, a detailed description of the invention may be had by reference to certain embodiments, some of which are illustrated in the accompanying drawings. It is to be noted, however, that the drawings illustrate only certain embodiments of this invention and are therefore not to be considered limiting of its scope, for the scope of the invention encompasses other equally effective embodiments. The drawings below are intended to be drawn neither to any precise scale with respect to relative size, angular relationship, relative position, or timing relationship, nor to any combinational relationship with respect to interchangeability, substitution, or representation of a required implementation, emphasis generally being placed upon illustrating the features of certain embodiments of the invention. In the drawings, like numerals are used to indicate like parts throughout the various views. Thus, for further understanding of the invention, reference can be made to the following detailed description, read in connection with the drawings in which:
FIG. 1A is a schematic diagram that shows a radiography apparatus for image acquisition and processing;
FIG. 1B is a schematic diagram that shows a mobile radiography apparatus that is provided on a movable cart;
FIG. 2 is a logic flow diagram that shows a processing sequence for generating a confidence map and related confidence factor according to one embodiment of the present disclosure;
FIGs. 3A and 3B are show examples of a residual image generated by combining pre-processed and processed image data;
FIG. 4 shows a confidence map generated by a computer system according to one embodiment; and
FIG. 5 shows an exemplary user interface displaying image content before and after processing and for closer examination of the residual image.

### DETAILED DESCRIPTION OF THE EMBODIMENTS

The following is a detailed description of the preferred embodiments, reference being made to the drawings in which the same reference numerals identify the same elements of structure in each of the several figures.

In the context of the present disclosure, the terms "image" and "image data" or "imaging data" are used equivalently to refer to the array of data pixels that can be displayed to show the image content.

The terminology "subject anatomy" or "subject" is considered equivalent in the context of the present disclosure, referring to the object of the optical system, wherein the optical system forms an image according to the exposure received by the object.

The term "highlighting" for a displayed feature has its conventional meaning as is understood to those skilled in the information and image display arts. In general, highlighting uses some form of localized display enhancement to attract the attention of the viewer to one or more particular portions of image content. Highlighting a portion of an image, such as a marker or an individual organ, bone, or structure, or a path from one chamber to the next, for example, can be achieved in any of a number of ways, including, but not limited to, annotating, displaying a nearby or overlaying symbol, outlining or tracing, display in a different color or at a markedly different intensity or gray scale value than other image or information content, blinking or animation of a portion of a display, or display at higher sharpness or contrast.

In typical applications, a computer or other type of dedicated logic processor for obtaining, processing, and storing image data is part of the radiography system, along with one or more displays for viewing image results. A computer-accessible memory is also provided, which may be a memory storage device used for longer term storage, such as a device using magnetic, optical, or other data storage media. In addition, the computer-accessible memory can comprise an electronic memory such as a random-access memory (RAM) that is used for shorter term storage, such as employed to store a computer program having instructions for controlling one or more computers to practice the method according to the present invention.

Aspects of the present disclosure are described primarily with reference to digital radiography (DR) system use. However, it can be readily appreciated that methods of the present disclosure can be readily adapted to other types of imaging systems, including those that acquire digital image data without the using of a DR detector, including computed radiography (CR) systems. In addition, embodiments of the present disclosure can apply to image data from other imaging types including ultrasound, (MRI), and projection image content from 3D volume imaging apparatus such as computed tomography (CT) or cone-beam computed tomography (CBCT) systems, for example.

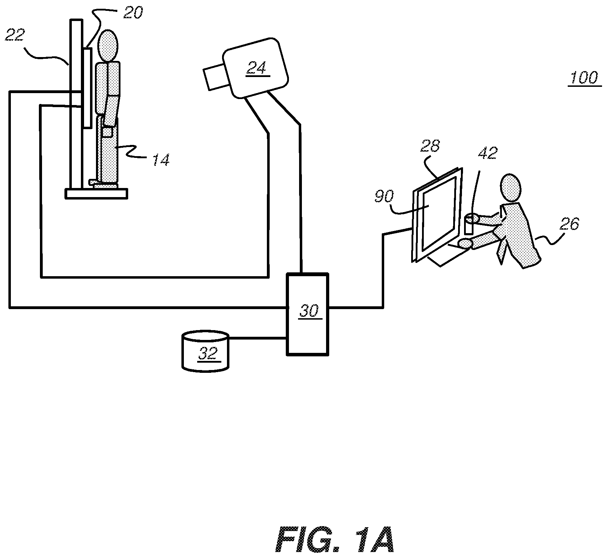

The schematic diagram of FIG. 1A shows a radiography apparatus 100 for in-room imaging. Apparatus 100 uses a DR detector 20 for image acquisition and provides computational logic for performing image processing on the acquired image and for analyzing results of the image processing for accuracy and faithfulness to the original image content and for reporting, storing, and transmitting these results. In the radiography apparatus 100 that is shown in FIG. 1A, a patient 14 is positioned adjacent DR detector 20, which is mounted on a bucky or other support 22, and is exposed to radiation from an x-ray source 24, whereby detector 20 captures a DR image of a portion of patient 14, for image acquisition. The exposure sequence is initiated by the technologist or other practitioner 26 using a manual operator control 42 that is in signal communication with a control logic processor 30 that can initiate exposure and the image acquisition and processing sequence described herein. Practitioner 26 controls the setup and operation from a workstation 28 having a display 90 or other control console protected from radiation scatter. Control logic processor 30 is in signal communication with other components of apparatus 100, such as DR detector 20 and x-ray source 24, and provides the needed control signals for exposure, data acquisition, processing, storage, and transmission. A memory 32 or other storage apparatus, in signal communication with control logic processor 30, can store the acquired image data.

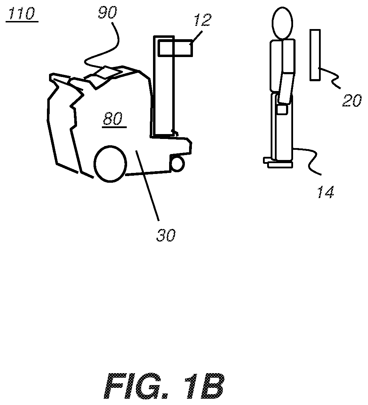

The schematic diagram of FIG. 1B shows an alternate embodiment of a mobile radiography apparatus 110 that is deployed for portable use on a cart 80 that supports an x-ray source 12 that directs radiation for imaging patient 14 using a wired or wireless DR detector 20. Cart 80 includes control logic processor 30 for acquisition control and on-board processing of the image content and presentation on display 90 or for wirelessly transmitting the acquired image data over a network to a networked processor (not shown) for subsequent image processing.

As noted previously in the background material, some amount of image pre-processing is executed automatically by acquisition hardware, firmware and/or software, in order to suitably condition the raw image data acquired from DR detector 20, according to calibration and hardware performance preprogrammed beforehand. The pre-processed image that is generated by this initial conditioning of the raw data can thus be considered as an "unprocessed" image; the raw data values generated within the DR detector require some measure of correction to condition the data due to varying characteristics inherent in the acquisition circuitry itself. Additional processing of data can then be applied to the pre-processed, conditioned image in order to suppress noise and to correct other undesirable aspects and, where useful, to enhance features of interest for viewing by the clinician or diagnostician or for subsequent analysis. Following this additional processing, embodiments of the present disclosure may provide added benefits in assisting the viewer to assess the overall quality of the additional imaging processing. Embodiments of the present disclosure can provide at least some amount of automated guidance to indicate the fidelity of the processed image to the original, unprocessed image by assigning a confidence factor or confidence rating to the processed image data. A localized confidence map can also indicate areas of the image that may be analyzed with corresponding levels of confidence.

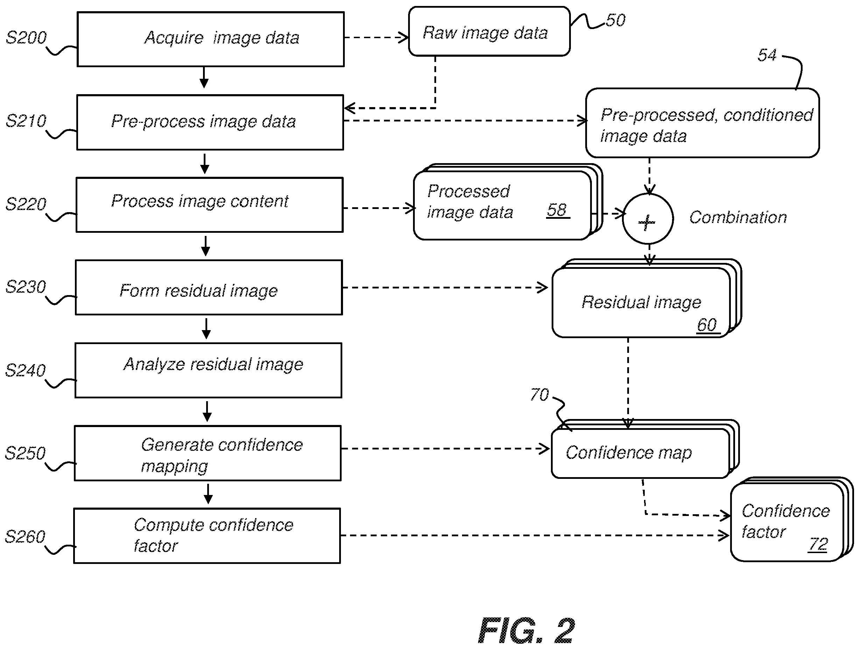

The logic flow diagram of FIG. 2 shows a method for image acquisition and processing according to one embodiment of the present disclosure. In an image acquisition step S200, the radiographic image is communicated (by wire or wirelessly) from the DR detector 20 (FIG. 1) as raw image data 50, typically read from an image buffer on the detector 20 or acquired as a stream of digital image data from data registers on the detector. A conditioning or pre-processing step S210 then provides initial processing of raw image data 50 that adjusts or conditions the data according to known calibration information obtained for detector 20 hardware and for the overall imaging system of the radiography apparatus 100 or 110. The calibration data can adjust for differences in pixel response and corresponding signals generated by the detector 20, including conditioning the data for pixels known to perform poorly, as identified beforehand during calibration procedures. Pre-processed, conditioned image data 54 is thereby generated.

The general type of pre-processing that is performed to condition the image content in step S210 is typically automatically executed, without operator input, and provides a conditioned image that faithfully represents subject features; however, the conditioned image data 54 may have visual characteristics that are less desirable, such as excessive noise or poor contrast, brightness, sharpness, or other characteristics. The pre-processed image formed from conditioned image data 54 can further include defects or artifacts, for example. A subsequent processing step S220 can then be executed to improve the visual appearance of the image and to enhance the clarity of features in the imaged anatomy, forming processed image data 58. Processing step S220 can perform various functions such as gain correction and adjustment, dark or offset calibration and/or correction, defect or artifact detection and correction, or other suitable image processing function.

As has been noted, methods of the present disclosure provide a mechanism for validating processed image data 58 and indicating the relative fidelity of image processing results, when evaluated against the original acquired and conditioned image data 54. To provide this function, a residual image generation step S230 executes, in which pre-processed, conditioned image data 54 and processed image data 58 are combined in order to generate a residual image 60. An analysis step S240 automatically analyzes residual image 60 in order to detect any differences in structure between the image data content for the two images, as these differences are exhibited in the generated residual image 60.

One or more confidence indicators can be provided by the logic sequence of FIG. 2. Results of analysis step S240 can be directed to a confidence mapping step S250 for generating a confidence map 70 that allows localized identification of suspect areas in the processed image. More generally, the analysis results can be directed to a confidence factor generation step S260 that generates a confidence factor 72. that applies for the processed image data 58 overall.

Portions of the FIG. 2 sequence are described in more detail in the following sections.

### Image processing in step S220

In general, the predominant type of image processing that is executed in processing step S220 of FIG. 2 relates to noise content in the acquired and conditioned image data 54. Noise typically appears as an irregular, granular or mottled pattern in the radiographic image and can degrade the quality of image information. Noise is predominantly related to exposure levels, with increased noise generated at lower exposures. During image acquisition, procedure techniques are followed by the technician in order to obtain an optimum exposure that generates an image having an acceptable noise level without unnecessary or excessive exposure to the patient.

Common sources of noise and factors related to noise levels in radiographic images can include electronic interference, digitization, quantum noise, scatter, detector sensitivity, absorption, and secondary radiation, for example.

Various algorithms have been developed to suppress noise in the acquired and conditioned image data without compromising the true image content. Typical noise suppression algorithms can employ various types of spatial or frequency-domain filters, configured to operate effectively to suppress random noise while having minimal impact on edges of image structures.

A widely acknowledged difficulty with noise suppression routines is that it can be difficult to distinguish random noise from true features in the image. For example, a set of noisy pixels can have similar characteristics to true edge transitions for anatomical features and lines, tubing, or instrumentation. Overly aggressive noise suppression can present the risk of degrading feature outlines or even compromising image data that relates to actual anatomy or features. An embodiment of the present disclosure follows the sequence of FIG. 2 in order to help identify the likelihood that image content is valid or may have been adversely affected by processing techniques.

Other exemplary types of image processing applied in step S220 to the pre-processed, conditioned image data 54 can include gain calibration and/or correction, dark or offset calibration and/or correction, scatter correction or compensation, rib or other bone suppression or enhancement, tone scale adjustment, and image defect identification and correction.

As is represented in FIG. 2, processing step S220 can optionally be repeated one or more times, or with different sets of variable parameters, in order to generate different versions of processed image data 58. This allows processing step S220 functions to be applied more or less aggressively to pre-processed, conditioned image data 54, giving the user the option to select the level of processing that is most appropriate for particular image content. Where multiple versions of processed image data 58 are generated, multiple corresponding residual images 60 can be formed by combining processed image data 58 with pre-processed conditioned image data 54 in step S230. Each residual image 60 can be indexed according to processing characteristics from corresponding processed image data 58, and can then be analyzed in analysis step S240 to generate a corresponding confidence map 70 and confidence factor 72.

### Forming the residual image in step S230

As is shown in the logic flow diagram of FIG. 2, residual image 60 can be formed by some combination of pre-processed, conditioned image 54 and processed image data 58. It can be appreciated that the combination process can be any operation that compares pixels of conditioned image 54 with corresponding pixels of processed image data 58 and provides an indication of the relative level of change between pixel values. According to an embodiment of the present disclosure, the combination process can be a straightforward subtraction of corresponding pixel values. Residual image 60 can then contain or represent, for each pixel position, the resulting difference.

Combination is expressed as a plus (+) sign in the FIG. 2 sequence; in practice, combination may involve addition or subtraction, with suitable weightings, or other operation that provides an image of pixel values according to relative pixel-by-pixel differences between two images of equivalent size. It should be noted that combination can involve any of a number of functions that facilitate comparing and operating upon the image data.

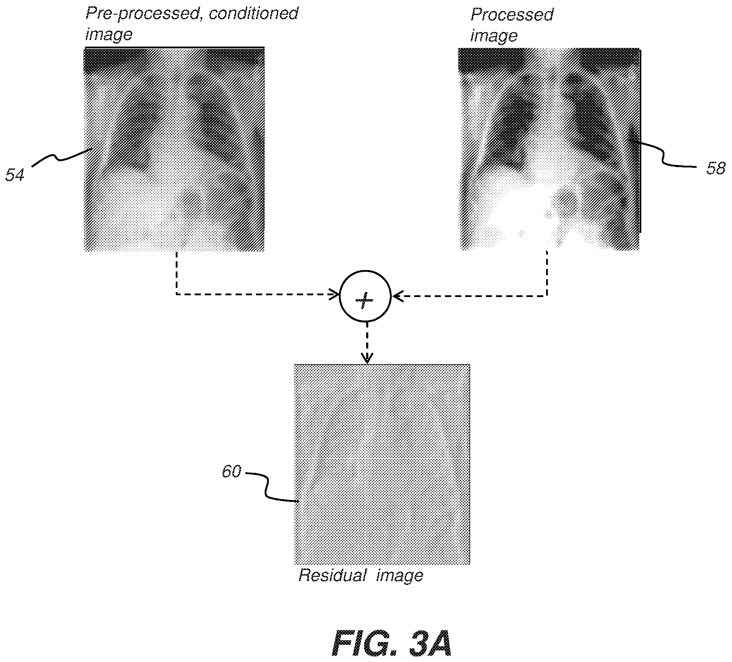

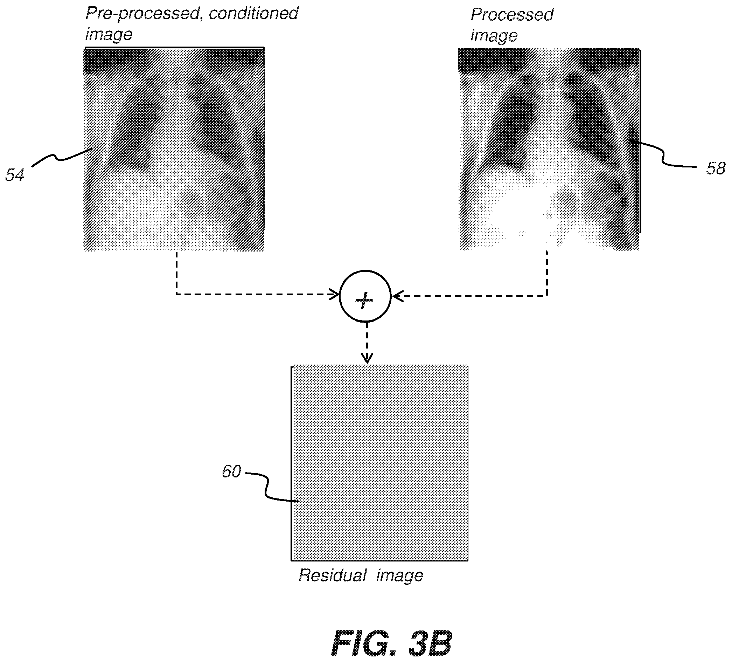

Other types of combination can alternately be used, including more complex combinations that process groupings of pixels or that show transitions between pixels in a more pronounced manner. This can include computing differential values between adjacent pixels in one or two dimensions, for example. Referring to the schematic representation of FIGs. 3A and 3B, generation of residual image using subtraction or other combination method is shown. In FIG. 3A, the resulting residual image 60 appears to indicate that processed image 58 and pre-processed, conditioned image 54 share equivalent information on image features, with moderate noise in the image content. In FIG. 3B, on the other hand, the resulting residual image 60 appears to indicate some level of difference between structural content of processed image 58 and pre-processed, conditioned image 54 with relation to the same image features.

### Analyzing the residual image in step S240

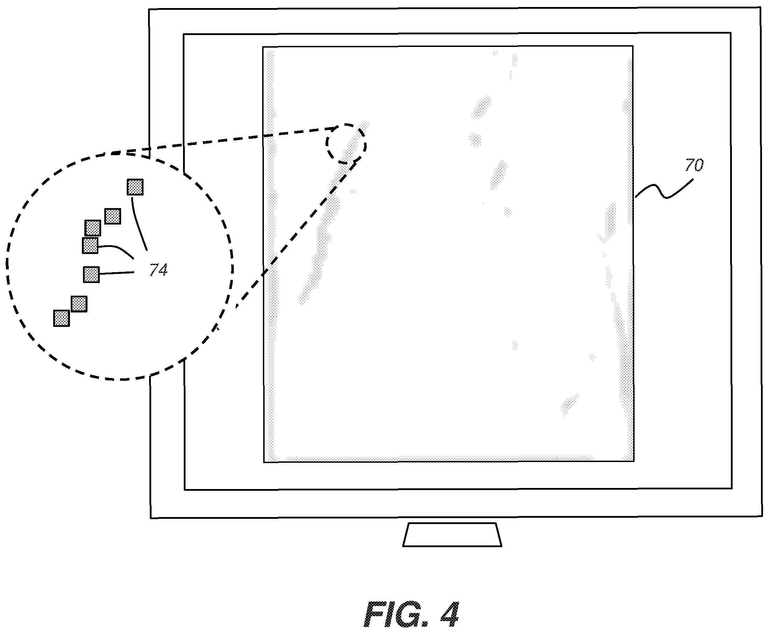

Further analysis and reporting of the relative fidelity of image processing can provide a confidence indicator that reports the computed results to a viewer. The schematic diagram of FIG. 4 shows a confidence map 70 that uses some form of localized highlighting to identify one or more portions of the residual image 60 that may have higher levels of change in image content due to image processing and, consequently, yield a lower confidence rating.

Analysis of the residual image 60 can include computing a standard deviation of noise or of values in the residual image.

Highlighting for confidence levels can be in the form of symbols 74, numbers, color, outlining, overlay, or other image treatment. As shown in FIG. 4, different colors, shading, or highlighting, can be used to indicate pixels or clusters of pixels within the residual image 70 that represent differences between processed image data 58 and pre-processed conditioned image data 54 above a threshold value that can be predefined for the processing software or that can be set and adjusted by a human viewer, and which may be used to indicate a lower confidence factor for image data in the highlighted portions. Localized confidence factors can be generated, such as for different portions of the residual image 70, based on the amount of difference between the pre-processed image data 54 and the processed image data 58. For example, a grid overlaid onto the residual image 60 or processed image 58 can display separate confidence factors for each cell within the grid.

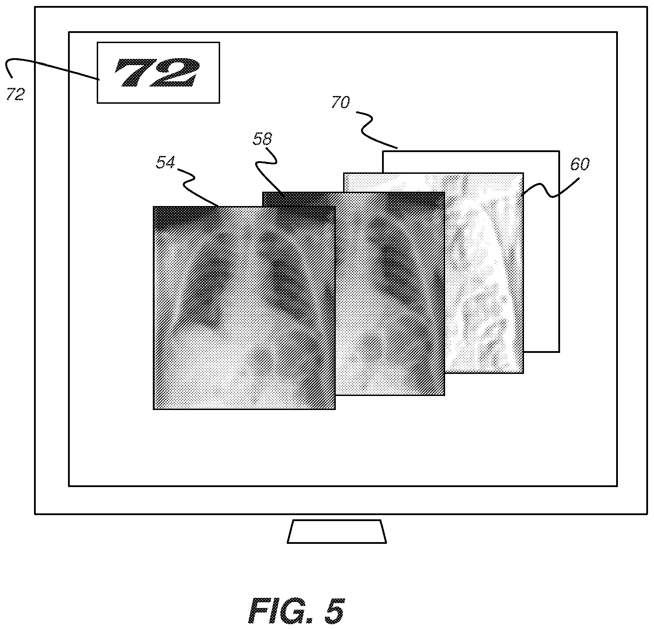

Alternately, a confidence factor 72 that applies to the full processed image can be displayed to the viewer, as is shown in the example of FIG. 5. Confidence factor 72. can be computed using an averaging process, such as a process that weights apparent features indicated by structure in the residual image 60, for example. Alternately, computation can generate an autocorrelation value or some other value indicative of image or pattern change.

FIG. 5 also shows an operator interface that allows the viewer to display and compare processed image data 58 with pre-processed, conditioned image data 54, which is shown in FIG. 5 as selectively brought to the foreground by a user, and to view residual image 60 and, optionally, confidence map 70. The viewer can click on the appropriate image in order to display that image in the foreground. The viewer can also selectively overlay confidence map 70 onto the residual image 60 or processed image 58.

Analysis of the residual image 60 can be used to determine a weighting or blending factor for combination of processed image data and pre-processed image data, for example.

According to an embodiment, the confidence rating can be presented as a graphic overlay over the processed image or over the pre-processed image, or both. The confidence rating can alternately be stored as part of a DICOM (Digital Imaging and Communications in Medicine) tag.

### Image set composition

According to an embodiment of the present disclosure, an image set can be formed, containing pre-processed, conditioned image data 54, processed image data 58, residual image data 60, and confidence map 70, with the optional addition or substitution of confidence factor 72 for map 70. An image set having this composition can be stored as a unit; alternately, links can be provided to different memory addresses or site locations for the various components of the image set. Image sets can thus be recalled for user viewing; each set including confidence data that can be useful for determining the relative accuracy and fidelity of the image processing that has been applied.

According to an embodiment of the present disclosure, there is a computer implemented method for processing a digital radiographic image, the method capturing and storing an unprocessed radiographic image acquired from a digital radiography (DR) detector. Image processing is performed on the unprocessed radiographic image and an image processed radiographic image is stored. The method combines the image processed radiographic image and the unprocessed radiographic image to form a residual image. The method further digitally analyzes the residual image to determine a confidence rating of the residual image and displays the determined confidence rating associated with the image processed radiographic image. The step of image processing can include one or more of gain calibration and/or correction, dark or offset calibration and/or correction, and defect identification and correction. The step of combining can include subtracting one of the processed radiographic image and the unprocessed, pre-conditioned radiographic image from the other. The step of digitally analyzing can include determining a standard deviation of noise in the residual image. The step of digitally analyzing can include analyzing the difference between the image-processed radiographic image and the unprocessed radiographic image. The step of digitally analyzing can include determining an auto correlation value in the residual image. The step of digitally analyzing can include determining an auto correlation value between the image processed radiographic image and the unprocessed radiographic image. The method can further include graphically overlaying the residual image onto the image processed radiographic image or the unprocessed radiographic image. The method can further include displaying the residual image for human visual analysis.

A computer implemented method for processing a digital radiographic image of a subject anatomy can include capturing and storing a pre-processed radiographic image acquired from a digital detector; repeating, for one or more iterations, a sequence of: (i) image processing the pre-processed radiographic image to form and store a processed radiographic image; (ii) combining the processed radiographic image and the pre-processed radiographic image to form a residual image; (iii) digitally analyzing the residual image to determine a confidence indicator that relates to the image processing corresponding to the iteration; and (iv) storing the processed image, the residual image, and the confidence indicator corresponding to the iteration in a memory; and recalling from the memory one or more of the stored processed image, residual image, and confidence indicator corresponding to a specified iteration; and displaying one or more of the recalled processed image, residual image, and confidence indicator in response to an operator selection. The method can further include storing an image set that links the pre-processed radiographic image acquired from the digital detector along with the processed radiographic image and a confidence indicator corresponding to the processed image of the subject anatomy.

A computer implemented method for processing a digital radiographic image, the method can include capturing and storing an unprocessed radiographic image acquired from a digital radiography (DR) detector; image processing the unprocessed radiographic image and storing the image processed radiographic image associated with the unprocessed radiographic image; combining the stored image processed radiographic image and the unprocessed radiographic image to form a residual image associated with the stored unprocessed and processed images; digitally analyzing the residual image to generate a confidence indicator related to fidelity of the image processed image to the unprocessed image; and displaying the generated confidence indicator associated with the image processed radiographic image. The method can further include associating the unprocessed image, the processed image, the residual image, and the corresponding confidence indicator for storage and recall. The method can further include associating the unprocessed image, the processed image, the residual image, and the corresponding confidence indicator for transmission. The method can further include simultaneously displaying the unprocessed image, the processed image, the residual image, and the corresponding confidence indicator on a display screen and responding to a viewer instruction to display the unprocessed image, the processed image, or the residual image at a larger size.

A computer program product may include one or more storage medium, for example; magnetic storage media such as magnetic disk (such as a floppy disk) or magnetic tape; optical storage media such as optical disk, optical tape, or machine readable bar code; solid-state electronic storage devices such as random access memory (RAM), or read-only memory (ROM); or any other physical device or media employed to store a computer program having instructions for controlling one or more computers to practice the method according to the present invention.

The invention has been described in detail, and may have been described with particular reference to a suitable or presently preferred embodiment, but it will be understood that variations and modifications can be effected within the scope of the invention. The presently disclosed embodiments are therefore considered in all respects to be illustrative and not restrictive. The scope of the invention is indicated by the appended claims.

## Claims

1. A computer (28) comprising a processing system (30) having stored therein a digital program configured to be executed by the processing system (30) to perform the steps of:
storing an unprocessed or pre-processed radiographic image (54) acquired from a digital radiographic detector (20);
image processing the unprocessed or pre-processed radiographic image (54) and storing the image processed radiographic image (58);
combining the image processed radiographic image (58) and the unprocessed or pre-processed radiographic image (54) to form a residual image (60), the residual image (60) indicating which image pixels, as between image pixels of the image processed radiographic image (58) and image pixels of the unprocessed or pre-processed radiographic image (54), have a greater level of change; and
digitally analyzing the residual image (60) to determine a numerical confidence rating of the residual image (60), whereby the image pixels having the greater level of change lower the numerical confidence rating;
wherein the processing system (30) is further configured to correct gain, offset, and defects in the unprocessed or pre-processed radiographic image (54).

2. The computer (28) of claim 1, wherein the processing system (30) is further configured to subtract one of the image processed radiographic image (58) and the unprocessed or pre-processed radiographic image (54) from the other.

3. The computer (28) of claim 1, wherein the processing system (30) is further configured to determine a standard deviation of noise as between the image processed radiographic image (58) and the unprocessed or pre-processed radiographic image (54).

4. The computer (28) of claim 1, wherein the processing system (30) is further configured to determine an auto correlation value in the residual image (60).

5. The computer (28) of claim 1, wherein the processing system (30) is further configured to determine an auto correlation value as between the image processed radiographic image (58) and the unprocessed or pre-processed radiographic image (54).

6. The computer (28) of claim 1, wherein the processing system (30) is further configured to graphically overlay the residual image (60) onto the image processed radiographic image (58) or the unprocessed or pre-processed radiographic image (54).

7. The computer (28) of claim 1, wherein the processing system (30) is further configured to display the residual image (60) for human visual analysis.

8. A computer implemented method for processing a digital radiographic image (54) of a subject anatomy, the method comprising:
capturing and storing (S200; S210) a pre-processed radiographic image (54) acquired from a digital detector (20);
iterating the steps of:
image processing (S220) and storing the pre-processed radiographic image (54) to form a processed radiographic image (58);
combining (S230) the processed radiographic image (58) and the pre-processed radiographic image (54) to form a residual image (60), the residual image (60) indicating which image pixels, as between image pixels of the processed radiographic image (58) and image pixels of the pre-processed radiographic image (54), have a greater level of change;
digitally analyzing (S240) the residual image (60)to determine (S260) a confidence indicator as between the pre-processed radiographic image (54) and the processed radiographic image (58), whereby the image pixels having the greater level of change decrease the confidence indicator; and
storing, in a computer-accessible memory (32), the processed image (58), the residual image (60), and the confidence indicator (72) corresponding to each of the iterations;
recalling from the memory one or more of the stored processed image, the residual image (60), and the confidence indicator of the corresponding iteration;
displaying one or more of the recalled processed image, the residual image (60), and the confidence indicator for a corresponding iteration in response to an operator request; and
performing only one of correcting gain, offset, or defects in each iteration.

9. The method of claim 8, further comprising:
storing an image set that links the pre-processed radiographic image (54) acquired from the digital detector along with the processed radiographic image (58) version corresponding to the pre-processed radiographic image (54); and
storing the confidence indicator corresponding to the processed radiographic image (58) version.

10. The method of claim 8, wherein the step of combining (S230) further comprises subtracting one of the image processed radiographic image (58) and the pre-processed radiographic image (54) from the other.

11. The method of claim 8, further comprising determining a standard deviation of noise as between the image processed radiographic image (58) and the pre-processed radiographic image (54).

12. The method of claim 8, further comprising graphically overlaying the residual image (60) onto the image processed radiographic image (58) or the pre-processed radiographic image (54).

13. The method of claim 8, further comprising simultaneously displaying the residual image (60), the image processed radiographic image (58), and the pre-processed radiographic image (54).

## Patentansprüche

1. Computer (28), der ein Verarbeitungssystem (30) umfasst, das ein darin gespeichertes digitales Programm aufweist, das dazu konfiguriert ist, durch das Verarbeitungssystem (30) ausgeführt zu werden, um die folgenden Schritte durchzuführen:
Speichern eines unverarbeiteten oder vorverarbeiteten radiografischen Bilds (54), das aus einem Digitalradiografiedetektor (20) erfasst wird;
Bildverarbeiten des unverarbeiteten oder vorverarbeiteten radiografischen Bilds (54) und Speichern des bildverarbeiteten radiografischen Bilds (58);
Kombinieren des bildverarbeiteten radiografischen Bilds (58) und des unverarbeiteten oder vorverarbeiteten radiografischen Bilds (54), um ein Residualbild (60) zu erzeugen, wobei das Residualbild (60) angibt, welche Bildpixel, wie zwischen Bildpixeln des bildverarbeiteten radiografischen Bilds (58) und Bildpixeln des unverarbeiteten oder vorverarbeiteten radiografischen Bilds (54), einen höheren Änderungsgrad aufweisen; und
digitales Analysieren des Residualbilds (60), um eine numerische Konfidenzbewertung des Residualbilds (60) zu bestimmen, wodurch die Bildpixel, die den höheren Änderungsgrad aufweisen, die numerische Konfidenzbewertung senken;
wobei das Verarbeitungssystem (30) ferner dazu konfiguriert ist, eine Verstärkung, einen Offset und Fehler in dem unverarbeiteten oder vorverarbeiteten radiografischen Bild (54) zu korrigieren.

2. Computer (28) nach Anspruch 1, wobei das Verarbeitungssystem (30) ferner dazu konfiguriert ist, eines von dem bildverarbeiteten radiografischen Bild (58) und dem unverarbeiteten oder vorverarbeiteten radiografischen Bild (54) von dem anderen zu subtrahieren.

3. Computer (28) nach Anspruch 1, wobei das Verarbeitungssystem (30) ferner dazu konfiguriert ist, eine Standardabweichung von Rauschen wie zwischen dem bildverarbeiteten radiografischen Bild (58) und dem unverarbeiteten oder vorverarbeiteten radiografischen Bild (54) zu bestimmen.

4. Computer (28) nach Anspruch 1, wobei das Verarbeitungssystem (30) ferner dazu konfiguriert ist, einen Autokorrelationswert in dem Residualbild (60) zu bestimmen.

5. Computer (28) nach Anspruch 1, wobei das Verarbeitungssystem (30) ferner dazu konfiguriert ist, einen Autokorrelationswert wie zwischen dem bildverarbeiteten radiografischen Bild (58) und dem unverarbeiteten oder vorverarbeiteten radiografischen Bild (54) zu bestimmen.

6. Computer (28) nach Anspruch 1, wobei das Verarbeitungssystem (30) ferner dazu konfiguriert ist, das Residualbild (60) auf dem bildverarbeiteten radiografischen Bild (58) oder dem unverarbeiteten oder vorverarbeiteten radiografischen Bild (54) grafisch einzublenden.

7. Computer (28) nach Anspruch 1, wobei das Verarbeitungssystem (30) ferner dazu konfiguriert ist, das Residualbild (60) zur menschlichen visuellen Analyse anzuzeigen.

8. Computerimplementiertes Verfahren zum Verarbeiten eines digitalen radiografischen Bilds (54) einer Patientenanatomie, wobei das Verfahren Folgendes umfasst:
Aufnehmen und Speichern (S200; S210) eines vorverarbeiteten radiografischen Bilds (54), das aus einem Digitaldetektor (20) erfasst wird;
Iterieren der folgenden Schritte:
Bildverarbeiten (S220) und Speichern des vorverarbeiteten radiografischen Bilds (54), um ein verarbeitetes radiografisches Bild (58) zu erzeugen;
Kombinieren (S230) des verarbeiteten radiografischen Bilds (58) und des vorverarbeiteten radiografischen Bilds (54), um ein Residualbild (60) zu erzeugen, wobei das Residualbild (60) angibt, welche Bildpixel, wie zwischen Bildpixeln des verarbeiteten radiografischen Bilds (58) und Bildpixeln des vorverarbeiteten radiografischen Bilds (54), einen höheren Änderungsgrad aufweisen;
digitales Analysieren (S240) des Residualbilds (60), um einen Konfidenzindikator wie zwischen dem vorverarbeiteten radiografischen Bild (54) und dem verarbeiteten radiografischen Bild (58) zu bestimmen (S260), wodurch die Bildpixel, die den höheren Änderungsgrad aufweisen, den Konfidenzindikator verringern; und
Speichern, in einem computerzugänglichen Speicher (32), des verarbeiteten Bilds (58), des Residualbilds (60) und des Konfidenzindikators (72), der mit jeder der Iterationen korrespondiert;
Abrufen, aus dem Speicher, eines oder mehrerer von dem gespeicherten verarbeiteten Bild, dem Residualbild (60) und dem Konfidenzindikator der korrespondierenden Iteration;
Anzeigen eines oder mehrerer von dem abgerufenen verarbeiteten Bild, dem Residualbild (60) und dem Konfidenzindikator für eine korrespondierende Iteration als Reaktion auf eine Bedieneranforderung; und
Durchführen nur eines beim Korrigieren einer Verstärkung, eines Offsets oder von Fehlern in jeder Iteration.

9. Verfahren nach Anspruch 8, das ferner Folgendes umfasst:
Speichern einer Bildmenge, die das vorverarbeitete radiografische Bild (54), das aus dem Digitaldetektor erfasst wird, zusammen mit der Version des verarbeiteten radiografischen Bilds (58), die mit dem vorverarbeiteten radiografischen Bild (54) korrespondiert, verknüpft; und
Speichern des Konfidenzindikators, der mit der Version des verarbeiteten radiografischen Bilds (58) korrespondiert.

10. Verfahren nach Anspruch 8, wobei der Schritt des Kombinierens (S230) ferner Subtrahieren eines von dem bildverarbeiteten radiografischen Bild (58) und dem vorverarbeiteten radiografischen Bild (54) von dem anderen umfasst.

11. Verfahren nach Anspruch 8, das ferner Bestimmen einer Standardabweichung von Rauschen wie zwischen dem bildverarbeiteten radiografischen Bild (58) und dem vorverarbeiteten radiografischen Bild (54) umfasst.

12. Verfahren nach Anspruch 8, das ferner grafisches Einblenden des Residualbilds (60) auf dem bildverarbeiteten radiografischen Bild (58) oder dem vorverarbeiteten radiografischen Bild (54) umfasst.

13. Verfahren nach Anspruch 8, das ferner gleichzeitiges Anzeigen des Residualbilds (60), des bildverarbeiteten radiografischen Bilds (58) und des vorverarbeiteten radiografischen Bilds (54) umfasst.

## Revendications

1. Ordinateur (28) comprenant un système de traitement (30) sur lequel est stocké un programme numérique configuré pour être exécuté par le système de traitement (30) pour réaliser les étapes consistant à :
stocker une image radiographique non traitée ou prétraitée (54) acquise auprès d'un détecteur radiographique numérique (20) ;
appliquer un traitement d'image à l'image radiographique non traitée ou prétraitée (54) et stocker l'image radiographique à traitement d'image (58) ;
combiner l'image radiographique à traitement d'image (58) et l'image radiographique non traitée ou prétraitée (54) pour former une image résiduelle (60), l'image résiduelle (60) indiquant quels pixels d'image, entre des pixels d'image de l'image radiographique à traitement d'image (58) et des pixels d'image de l'image radiographique non traitée ou prétraitée (54), présentent un niveau de variation supérieur ; et
analyser numériquement l'image résiduelle (60) pour déterminer une note de confiance chiffrée de l'image résiduelle (60), ce par quoi les pixels d'image qui présentent le niveau de variation supérieur abaissent la note de confiance chiffrée ;
le système de traitement (30) étant en outre configuré pour corriger le gain, le décalage et les défauts dans l'image radiographique non traitée ou prétraitée (54).

2. Ordinateur (28) selon la revendication 1, le système de traitement (30) étant en outre configuré pour soustraire l'une de l'image radiographique à traitement d'image (58) et de l'image radiographique non traitée ou prétraitée (54) de l'autre.

3. Ordinateur (28) selon la revendication 1, le système de traitement (30) étant en outre configuré pour déterminer un écart-type de bruit entre l'image radiographique à traitement d'image (58) et l'image radiographique non traitée ou prétraitée (54).

4. Ordinateur (28) selon la revendication 1, le système de traitement (30) étant en outre configuré pour déterminer une valeur d'autocorrélation dans l'image résiduelle (60).

5. Ordinateur (28) selon la revendication 1, le système de traitement (30) étant en outre configuré pour déterminer une valeur d'autocorrélation entre l'image radiographique à traitement d'image (58) et l'image radiographique non traitée ou prétraitée (54).

6. Ordinateur (28) selon la revendication 1, le système de traitement (30) étant en outre configuré pour superposer graphiquement l'image résiduelle (60) sur l'image radiographique à traitement d'image (58) ou sur l'image radiographique non traitée ou prétraitée (54).

7. Ordinateur (28) selon la revendication 1, le système de traitement (30) étant en outre configuré pour afficher l'image résiduelle (60) en vue d'une analyse visuelle humaine.

8. Procédé mis en œuvre par ordinateur pour traiter une image radiographique numérique (54) d'une anatomie de sujet, le procédé comprenant les étapes consistant à :
capturer et stocker (S200 ; S210) une image radiographique prétraitée (54) acquise auprès d'un détecteur numérique (20) ;
itérer les étapes consistant à :
appliquer un traitement d'image (S220) à l'image radiographique prétraitée (54) et la stocker pour former une image radiographique traitée (58) ;
combiner (S230) l'image radiographique traitée (58) et l'image radiographique prétraitée (54) pour former une image résiduelle (60), l'image résiduelle (60) indiquant quels pixels d'image, entre des pixels d'image de l'image radiographique traitée (58) et des pixels d'image de l'image radiographique prétraitée (54), présentent un niveau de variation supérieur ;
analyser numériquement (S240) l'image résiduelle (60) pour déterminer (S260) un indicateur de confiance entre l'image radiographique prétraitée (54) et l'image radiographique traitée (58), ce par quoi les pixels d'image qui présentent le niveau de variation supérieur diminuent l'indicateur de confiance ; et
stocker, dans une mémoire accessible par ordinateur (32), l'image traitée (58), l'image résiduelle (60) et l'indicateur de confiance (72) correspondant à chacune des itérations ;
rappeler, de la mémoire, un ou plusieurs éléments parmi l'image traitée stockée, l'image résiduelle (60) et l'indicateur de confiance de l'itération correspondante ;
afficher un ou plusieurs éléments parmi l'image traitée rappelée, l'image résiduelle (60) et l'indicateur de confiance pour une itération correspondante en réponse à une demande d'opérateur ; et
réaliser une seule correction parmi une correction du gain, du décalage ou des défauts dans chaque itération.

9. Procédé selon la revendication 8, comprenant en outre les étapes consistant à :
stocker un ensemble d'images qui lie l'image radiographique prétraitée (54) acquise auprès du détecteur numérique à la version de l'image radiographique traitée (58) correspondant à l'image radiographique prétraitée (54) ; et
stocker l'indicateur de confiance correspondant à la version de l'image radiographique traitée (58).

10. Procédé selon la revendication 8, l'étape de combinaison (S230) comprenant en outre l'étape consistant à soustraire l'une de l'image radiographique à traitement d'image (58) et de l'image radiographique prétraitée (54) de l'autre.

11. Procédé selon la revendication 8, comprenant en outre l'étape consistant à déterminer un écart-type de bruit entre l'image radiographique à traitement d'image (58) et l'image radiographique prétraitée (54).

12. Procédé selon la revendication 8, comprenant en outre l'étape consistant à superposer graphiquement l'image résiduelle (60) sur l'image radiographique à traitement d'image (58) ou sur l'image radiographique prétraitée (54).

13. Procédé selon la revendication 8, comprenant en outre l'étape consistant à afficher simultanément l'image résiduelle (60), l'image radiographique à traitement d'image (58) et l'image radiographique prétraitée (54).
